# EUROPEAN PATENT APPLICATION

(11) **EP 3 940 062 A1**
(43) Date of publication of application: **19.01.2022**
(21) Application number: 20798513.6
(22) Date of filing: 27.04.2020
(51) Int. Cl.: C12N 5/0775, A61K 8/98, A61Q 19/08, A61Q 19/02, A61Q 7/00

(54) **COSMETIC COMPOSITION COMPRISING CULTURE SOLUTION OF MESENCHYMAL STEM CELLS CULTURED IN HPL-CONTAINING MEDIUM**

(30) Priority: 02.05.2019 KR 20190051596
(71) Applicant: SCM Lifescience Co., Ltd., Jung-gu Incheon 22332 (KR)
(72) Inventor: SONG, Sun Uk, Incheon 22003 (KR); KIM, Si Na, Incheon 21997 (KR); LEE, Chan Ju, Incheon 21997 (KR)
(74) Representative: Altmann Stößel Dick Patentanwälte PartG mbB
(86) International application number: PCT/KR2020/005483
(87) International publication number: WO 2020/222472

(57) **Abstract**

The present invention relates to: a method for preparing a stem cell culture solution which contains growth factors and cytokines and is obtained by culturing, in a serum-free medium, mesenchymal stem cells cultured in a medium containing human platelet lysates (hPLs); and a cosmetic composition using the stem cell culture solution. When the method for preparing a mesenchymal stem cell culture solution obtained from mesenchymal stem cells cultured in an hPL-containing medium is used, it is possible to obtain a mesenchymal stem cell culture solution which has a higher amount of cytokines and growth factors than that of a mesenchymal stem cell culture solution obtained from mesenchymal stem cells cultured in an FBS-containing medium, and has excellent efficacy in the promotion of human dermal fibroblast proliferation, collagen synthesis, elastin synthesis, inhibition of melanin synthesis, and hair growth. The mesenchymal stem cell culture solution can be used to produce cosmetics having effects of skin regeneration, wrinkle improvement, skin elasticity enhancement, skin whitening, and hair growth, and thus can be widely used in the cosmetic field and the pharmaceutical industry.

## Description

### [Technical Field]

The present invention relates to a method for producing a stem cell culture solution containing growth factors and cytokines obtained by culturing mesenchymal stem cells cultured in a medium containing human platelet lysate (hPL) in a serum-free medium, and the stem cell culture solution and a cosmetic composition using the stem cell culture solution.

### [Background Art]

Skin aging is largely divided into two types: one is "internal aging," which is an aging phenomenon caused by increasing age, and the other is "external aging," which refers to aging caused by external environments such as UV rays or pollution. Several theories have been suggested about skin aging, but among them, the most scientifically approached theory on skin aging is the theory in which skin aging is caused by oxidation. Human skin is composed of the epidermis including the stratum corneum, dermis, and connective tissue. Among them, the stratum corneum is composed of a dead cell layer formed through the differentiation process of keratinocytes, the basal cells of the epidermis, and plays a role in protecting the human body from the influence of the external environment. Further, the dermis layer existing inside the skin is composed of collagen and elastin and plays a role in giving the skin elasticity to protect the skin from sagging. The antioxidant theory is the theory that collagen and elastin are damaged by free radicals generated by external factors such as ultraviolet rays, and accordingly, the skin elasticity is reduced, and wrinkles are generated.

Until now, substances such as retinol or plant extracts have been used to prevent such skin aging, in particular wrinkles. However, these substances have a disadvantage in that their effectiveness is low, or they have serious side effects on the skin, inducing skin irritation, redness, and inflammation.

A person's skin color is determined by the concentration and distribution of melanin inside the skin, which is also affected by environmental conditions such as sun ultraviolet rays or physiological conditions such as fatigue and stress in addition to genetic factors. Melanin is produced through a non-enzymatic oxidation reaction after the enzyme tyrosinase acts on tyrosine, a kind of amino acid, to be converted into DOPA and dopaquinone. When such melanin synthesis occurs in the skin excessively, the skin tone is darkened, and spots, freckles, and the like may occur. Therefore, the synthesis of melanin in the skin is inhibited not only to realize skin whitening by brightening the skin tone but also to improve skin hyperpigmentation such as spots and freckles caused by ultraviolet rays, hormones, and genetic causes. Conventionally, substances having inhibitory activity against tyrosinase, such as hydroquinone, ascorbic acid, kojic acid, and glutathione, are mixed and used in ointments or cosmetics to improve skin whitening or alleviating spots and freckles. Among them, hydroquinone has been used as the most effective ingredient for pigmentation for decades, but there are reports that side effects (white spots and black spots) such as allergic reactions may occur with long-term use. Thus, there is a trend towards restrictions on its use in several countries. Thiol-based compounds such as glutathione and cysteine have a problem in their use due to their characteristic unpleasant odor and a problem of percutaneous absorption. Further, these glycosides and derivatives have high polarity, so there is a problem in being used as a formulation component of cosmetics.

Further, ascorbic acid inhibits the activity of the tyrosinase enzyme to exhibit a whitening effect, but it is easily oxidized. Thus, cosmetics containing it cause problems such as a change in color and odor. Derivatives such as ascorbyl palmitate, ascorbyl-dipalmitate, ascorbyl stearate, and ascorbyl magnesium phosphate have been developed and used to overcome the instability. However, it has the disadvantage that unless special technology is used, it is difficult to penetrate into the skin and tyrosinase inhibitory activity is low. Therefore, the development of inhibitors capable of exhibiting tyrosinase inhibitory activity even in a small amount and inhibiting melanin biosynthesis is required.

### [Disclosure]

### [Technical Problem]

Accordingly, the present inventors made diligent efforts to develop a cosmetic with excellent anti-wrinkle effect, whitening effect and hair growth effect, thereby confirming that compared to the stem cell culture solution obtained by culturing stem cells in FBS-containing medium, the stem cell culture solution obtained by culturing stem cells in an hPL-containing medium is rich in growth factors and cytokines and has excellent human dermal fibroblast proliferation promoting ability, collagen synthesis ability, elastin synthesis ability, melanin synthesis inhibitory ability and hair growth promoting ability to have an excellent effect as a functional cosmetic, thereby completing the present invention.

Accordingly, an object of the present invention provides a method for producing a mesenchymal stem cell culture solution rich in growth factors and cytokines, a mesenchymal stem cell culture solution prepared by the above method, a cosmetic composition for skin regeneration including the mesenchymal stem cell culture solution, a cosmetic composition for wrinkle improvement and skin elasticity enhancement including a mesenchymal stem cell culture solution, a cosmetic composition for skin whitening including the mesenchymal stem cell culture solution, and a cosmetic composition for promoting hair growth or hair increase including the mesenchymal stem cell culture solution.

### [Technical Solution]

In order to achieve the above object, the present invention provides a method for producing a mesenchymal stem cell culture solution rich in growth factors and cytokines, the method including steps of: 1) culturing mesenchymal stem cells in a medium containing human platelet lysate (hPL); 2) obtaining the cultured stem cell and culturing the stem cells in a serum-free medium; and 3) collecting and filtering the mesenchymal stem cell-derived culture solution to obtain a stem cell culture solution.

Further, the present invention provides a method for producing a mesenchymal stem cell culture solution rich in growth factors and cytokines for preparing a cosmetic composition for skin regeneration, wrinkle improvement, skin whitening, hair growth, the method including steps of: 1) culturing the mesenchymal stem cells in a medium containing human platelet lysate (hPL); 2) obtaining cultured stem cells and culturing them in a serum-free medium; and 3) collecting and filtering the mesenchymal stem cell-derived culture solution to obtain a stem cell culture solution.

Further, the present invention provides a mesenchymal stem cell culture solution rich in growth factors and cytokines prepared by the method.

Further, the present invention provides a cosmetic composition for use in skin regeneration including the mesenchymal stem cell culture solution.

Further, the present invention provides a cosmetic composition for use in wrinkle improvement and skin elasticity enhancement including the mesenchymal stem cell culture solution.

Further, the present invention provides a cosmetic composition for use in skin whitening including the mesenchymal stem cell culture solution.

Further, the present invention provides a cosmetic composition for use in hair growth or hair increase promotion including the mesenchymal stem cell culture solution.

### [Advantageous Effects]

Compared to the mesenchymal stem cell culture solution in which the mesenchymal stem cells are cultured in the FBS-containing medium, the method for producing a mesenchymal stem cell culture solution in which the mesenchymal stem cells are cultured in the hPL-containing medium of the present invention is used to obtain the mesenchymal stem cells having a higher amount of cytokines and growth factors and excellent human dermal fibroblast proliferation promoting ability, collagen synthesis ability, elastin synthesis ability, melanin synthesis inhibitory ability and hair growth efficacy. Thus, the mesenchymal stem cell culture solution is used in cosmetics for skin regeneration, wrinkle improvement, skin elasticity enhancement, skin whitening and hair growth effects so that it can be widely used in cosmetics and pharmaceutical industries.

### [Description of Drawings]

FIG. 1 is a view showing an array map of a commercially available assay kit (proteome profiler human XL cytokine array kit, R&D).
FIG. 2A illustrates a result of analyzing the protein and growth factors present in the stem cell culture solution according to the culture conditions: Con-media refers to serum-free DMEM cultured in cell-free conditions, FBS-CM refers to mesenchymal stem cell culture solution cultured in 10% FBS condition, and hPL-CM refers to mesenchymal stem cell culture solution cultured in 4% hPL condition. The solid square box with the thick solid line represents the experimental control, and the square box with the thin solid line represents the protein detected only in the hPL condition.
FIG. 2B is a view showing a protein expressed only in hPL culture conditions.
FIG. 2C is a view confirming the expression of the growth factor in the stem cell culture solution.
FIG. 2D is a view confirming the expression of a protein having a skin rejuvenation effect in the stem cell culture solution.
FIG. 2E is a view confirming the expression of stem cell efficacy markers, migration or regeneration-related proteins.
FIG. 2F is a view confirming the protein expressed in hPL culture conditions, although not classified in FIGS 2B to 2E.
FIG. 3 is a view showing comparative analysis of the amounts of VEGF, TGF-beta1, HGF, and FGF, which are representative major growth factors present in the stem cell culture solution according to the culture conditions.
FIG. 4 is a view showing a comparison of the proliferation promoting effect of human dermal fibroblasts according to the culture conditions.
FIG. 5 is a view showing a comparison of the collagen synthesis effect according to the culture conditions.
FIG. 6 is a view showing a comparison of the elastin synthesis effect according to the culture conditions.
FIG. 7 is a view showing a comparison of skin whitening effects according to culture conditions using 3D artificial skin.
FIG. 8 is a view showing a comparison of the proliferation promoting effect in human papilla cells according to the culture conditions.
FIG. 9 is a view showing a comparison of the proliferation promoting effect of human dermal fibroblasts according to the culture conditions and the separation method.
FIG. 10 is a view showing a comparison of the amount of VEGF according to the culture conditions and the separation method.

### [Modes of the Invention]

The present invention provides a method for producing a mesenchymal stem cell culture solution rich in growth factors and cytokines, the method including steps of: 1) culturing mesenchymal stem cells in a medium containing human platelet lysate (hPL); 2) obtaining the cultured stem cell and culturing the stem cells in a serum-free medium; and 3) collecting and filtering the mesenchymal stem cell-derived culture solution to obtain a stem cell culture solution.

In the present invention, the term "human platelet lysate (hPL)" is a turbid, pale yellow liquid obtained after freezing and thawing human platelets. Platelets are frozen and thawed to be lysed, thereby releasing large amounts of growth factors necessary for cell expansion.

In the present invention, the term "stem cell culture solution" refers to a substance containing components contained in a medium obtained by culturing stem cells. The mesenchymal stem cells for preparing the culture solution are not limited in type. It may be stem cells derived from cartilage, bone tissue, adipose tissue and bone marrow, preferably mesenchymal stem cells derived from bone marrow.

The method for producing the mesenchymal stem cell culture solution of the present invention may be characterized by using the stem cell culture solution obtained by culturing the cells in an hPL-containing medium of "step 1)" and culturing the cells in a serum-free medium of "step 2)."

Since serum and antibiotics are not included in the stem cell culture solution obtained by the above two-step process, a stem cell culture solution suitable for a cosmetic composition may be obtained.

As the medium for culturing stem cells in the present invention, a basal medium known in the art may be used without limitation. The basal medium may be artificially synthesized and manufactured, or a commercially prepared medium may be used. Examples of commercially prepared medium include Dulbecco's modified eagle's medium (DMEM), minimal essential medium (DMEM), basal medium eagle (BME), RPMI 1640, F-10, F-12, α-minimal essential medium (α-MEM), Glasgow's minimal essential medium (G-MEM), and Isocove's modified Dulbecco's medium, but are not limited thereto.

The medium used in the present invention may include a medium without an antioxidant, a medium supplemented with an antioxidant, or a medium containing an antioxidant.

The medium without an antioxidant may, but be not limited to, include DMEM. If necessary, an antioxidant may further be added to the medium to perform the culture. If necessary, α-MEM containing an antioxidant may be used to perform the culture.

The antioxidants of the present invention may include, without limitation, antioxidants that can be used in cell cultures. They may include one or more selected from the group consisting of glutathione, cysteine, cysteamine, ubiquinol, betamercaptoethanol and ascorbic acid. When the medium is supplemented with an antioxidant, the antioxidant may be added at a concentration of 10 to 50 µg/ml, preferably 10 to 30 µg/ml, and more preferably 25 µg/ml.

In one embodiment of the present invention, DMEM, more preferably LG-DMEM, is used as a medium without an antioxidant, and α-MEM is used as a medium containing ascorbic acid as an antioxidant.

In the present invention, the mesenchymal stem cells of "step 1)" are mesenchymal stem cells separated by a gradient centrifugation method (GCM) or subfractionation culturing method (SCM) (Korean Application No. 10-2006-0075676).

In the present invention, the mesenchymal stem cell culture solution prepared by using the mesenchymal stem cells separated by the subfractionation culturing method from the "mesenchymal stem cells of step 1)" has a higher amount of growth factor and cytokine and has excellent skin regeneration, wrinkle improvement, skin whitening, hair growth or hair increase effects compared to the mesenchymal stem cell culture solution prepared by using the mesenchymal stem cells separated by the gradient centrifugation method (GCM) from the "mesenchymal stem cells of step 1)."

The "subfractionation culturing method" refers to a method of separating stem cells according to specific gravity. First, the bone marrow is extracted from the subject and cultured in a cell culture solution. Only the supernatant is collected, and this is transferred to a culture vessel treated with or without a coating agent to be cultured. Then, the same process is repeated several times. The method may preferably be a method including steps of: 1) culturing the bone marrow isolated from the subject; 2) transferring only the supernatant from step 1) to a new vessel and culturing the same; 3) separating only the supernatant from step 2), then culturing the supernatant for 1 hour to 3 hours at 35°C to 39°C in a culture vessel, then repeating the culture for 12 to 36 hours at 35°C to 39°C 2 to 3 times, then culturing for 24 hours to 72 hours at 35°C to 39°C, in which each time transferring the supernatant to a new culture vessel and repeating the culture; and 4) obtaining monoclonal stem cells from the final culture vessel.

In the present invention, the culturing of "step 1)" may be a method including; inoculating and culturing mesenchymal stem cells in a medium containing hPL at a cell density of 10 to 10,000 cells/cm² and preferably, inoculating and culturing the mesenchymal stem cells in a medium at a cell density of 50 to 1,000 cells/cm².

In the present invention, the medium of "step 1)" may contain 0.1% to 20% (w/w) of human platelet lysate (hPL), preferably 1% to 10% (w/w) of hPL.

In the present invention, the mesenchymal stem cell culture solution prepared by using an hPL-containing medium as the medium of "step 1)" has a higher growth factor and cytokine amount, and has excellent skin regeneration, wrinkle improvement, skin whitening, and hair growth or hair increase effects compared to a mesenchymal stem cell culture solution prepared by using an FBS-containing medium as the medium of "step 1)."

In the present invention, "'growth factors and cytokines" may be any growth factors, cytokines or proteins expressed by mesenchymal stem cells, preferably any one or more, two or more, three or more, four or more, five or more, six or more, seven or more, eight or more, nine or more, ten or more, eleven or more, twelve or more, thirteen or more, fourteen or more, fifteen or more, sixteen or more, seventeen or more, eighteen or more, nineteen or more, twenty or more, twenty one or more, twenty two or more, twenty three or more, twenty four or more, twenty five or more, twenty six or more, twenty seven or more, twenty eight or more, twenty nine or more, thirty or more, thirty one or more, thirty two or more, thirty three or more, thirty four or more, thirty five or more, thirty six or more, thirty seven or more, thirty eight or more, thirty nine or more, forty or more, forty one or more, forty two or more, forty three or more, forty four or more, forty five or more, forty six or more, forty seven or more, or forty-eight or more selected from the group consisting of apolipoprotein-A-1, chemokine (C-X-C motif) ligand-4 (CXCL-4), chemokine (C-C motif) ligand-5 (CCL-5), retinol binding protein-4 (RBP-4), urokinase receptor (uPAR), vitamin D-BP, vascular endothelial growth factor (VEGF), fibroblast growth factor 2 (FGF2), hepatocyte growth factor (HGF), fibroblast growth factor 7 (FGF7), fibroblast growth factor 19 (FGF19), angiogenin, angiopoietin-1, angiopoietin-2, Dickoff related protein 1 (DKK-1), insulin like growth factor binding protein-2 (IGFBP-2), interleukin-6 (IL-6), interleukin-8 (IL -8), platelet derived growth factor-AA (PDGF-AA), macrophage colony stimulating factor (M-CSF), insulin like growth factor binding protein-3 (IGFBP-3), vascular cell adhesion molecule-1 (VCAM-1), leukemia inhibitory factor (LIF), chemokine (C-X-C motif) ligand-12 (CXCL-12), chemokine (C-C motif) ligand-2 (CCL-2), pentraxin-3, chemokine (C-C motif) ligand-7 (CCL-7), adiponectin, Fas ligand (TNF SF6), macrophage migration inhibitory factor (MIF), interleukin-22 (IL-22), interleukin-4 (IL-4), interleukin-17A (IL-17A), complement component 5/complement component 5a (C5/C5a), cluster of differentiation 14 (CD14), chitinase 3-like 1, adipsin, C-reactive protein, dipeptidyl peptidase IV (DPP IV), cystatin C, emprin, growth/differentiation factor 15 (GDF15), osteopontin, serpin E1 (nexin), thrombospondin-1 (TSP-1), resistin, brain derived neurotrophic factor (BDNF) and endoglin. More preferably, "'growth factors and cytokines" may be any one or more, two or more, three or more, four or more, five or more, six or more, seven or more, eight or more, nine or more, ten or more, eleven or more, twelve or more, thirteen or more, fourteen or more, fifteen or more, or sixteen or more selected from the group consisting of apolipoprotein-A-1, chemokine (C-X-C motif) ligand-4 (CXCL-4), chemokine (C-C motif) ligand-5 (CCL-5), retinol binding protein-4 (RBP-4), vitamin D-BP, adiponectin, brain derived neurotrophic factor (BDNF), complement component 5/complement component 5a (C5/C5a), cluster of differentiation 14 (CD14), Fas ligand (TNF SF6), growth/differentiation factor 15 (GDF15), interleukin-6 (IL-6), interleukin-8 (IL-8), platelet derived growth factor-AA (PDGF-AA), macrophage colony stimulating factor (M-CSF) and leukemia inhibitory factor (LIF), which are specifically present in the mesenchymal stem cell culture solution. The culture solution is obtained by culturing the mesenchymal stem cells in hPL-containing medium and obtaining the stem cells, followed by culturing the obtained stem cells in a serum-free medium. More preferably, "'growth factors and cytokines" may further include any one or more, two or more, three or more, four or more, five or more, six or more, seven or more, eight or more, nine or more, ten or more, eleven or more, twelve or more, thirteen or more, fourteen or more, fifteen or more, sixteen or more, seventeen or more, eighteen or more, nineteen or more, twenty or more, twenty one or more, twenty two or more, twenty three or more, twenty four or more, twenty five or more, twenty six or more, twenty seven or more, twenty eight or more, twenty nine or more, thirty or more, thirty one or more, or thirty two or more selected from the group consisting of vascular endothelial growth factor (VEGF), fibroblast growth factor 2 (FGF2), hepatocyte growth factor (HGF), fibroblast growth factor 7 (FGF7), fibroblast growth factor 19 (FGF19), angiogenin, angiopoietin-1, angiopoietin-2, Dickoff related protein 1 (DKK-1), insulin like growth factor binding protein-2 (IGFBP-2), insulin like growth factor binding protein-3 (IGFBP-3), vascular cell adhesion molecule-1 (VCAM-1), chemokine (C-X-C motif) ligand-12 (CXCL-12), chemokine (C-C motif) ligand-2 (CCL-2), pentraxin-3, chemokine (C-C motif) ligand-7 (CCL-7), macrophage migration inhibitory factor (MIF), interleukin-22 (IL-22), interleukin-4 (IL-4), interleukin-17A (IL-17A), chitinase 3-like 1, adipsin, C-reactive protein, dipeptidyl peptidase IV (DPP IV), cystatin C, emprin, osteopontin, serpin E1 (nexin), thrombospondin-1 (TSP-1), resistin, urokinase receptor (uPAR) and endoglin, which have a significant increase in amount in the mesenchymal stem cell culture solution by culturing the mesenchymal stem cells in hPL-containing medium and obtaining the stem cells, followed by culturing the obtained stem cells in a serum-free medium again, compared to the mesenchymal stem cell culture solution obtained by culturing the mesenchymal stem cells in a medium containing FBS and obtaining the stem cells, followed by culturing the obtained stem cells in a serum-free medium again.

Further, the present invention provides a method for producing a mesenchymal stem cell culture solution rich in growth factors and cytokines for preparing a cosmetic composition for skin regeneration, wrinkle improvement, skin whitening, hair growth or hair increase, the method including steps of: 1) culturing the mesenchymal stem cells in a medium containing human platelet lysate (hPL); 2) obtaining cultured stem cells and culturing them in a serum-free medium; and 3) collecting and filtering the mesenchymal stem cell-derived culture solution to obtain a stem cell culture solution.

In the present invention, "skin regeneration" may refer to any action that replenishes a part of the skin or promotes the proliferation of skin cells when a part of the skin is lost. When the proliferation of dermal fibroblasts is promoted, the skin regeneration effect may appear. The mesenchymal stem cell culture solution for preparing the cosmetic composition of the present invention has an excellent proliferation promoting effect on human dermal fibroblasts, and has an excellent skin regeneration effect.

In the present invention, "wrinkle improvement" refers to maintaining or enhancing the skin's ability related to wrinkle and elasticity. Among the structures of the skin, collagen fibers (collagen) and elastic fibers (elastin) present in the dermis layer are the main proteins that play the role. They control skin elasticity, and the biosynthesis of collagen is affected internally and externally. The mesenchymal stem cell culture solution for preparing the cosmetic composition of the present invention has excellent collagen synthesis ability and elastin synthesis ability and has an excellent wrinkle improvement effect.

In the present invention, "whitening" refers to the effect of brightening skin color. The skin color is determined by the amount of melanin that is synthesized in melanocytes. The mesenchymal stem cell culture solution for preparing the cosmetic composition of the present invention reduces the synthesis of melanin pigment, and thus has an excellent skin whitening effect.

In the present invention, "hair growth" refers to hair growth from the scalp, "hair increase" refers to lengthening of hair (i.e., hair growth), and has the same meaning as "hair development" another term used in the art. The hair growth or hair increase effect is promoted by the generation or proliferation of dermal papilla cells, and the mesenchymal stem cell culture solution for preparing the cosmetic composition of the present invention promotes the proliferation of dermal papilla cells, and thus has excellent hair growth and hair increase effects.

In addition, the present invention provides a mesenchymal stem cell culture solution rich in growth factors and cytokines prepared by the above production method.

The mesenchymal stem cell culture solution of the present invention promotes the proliferation of dermal fibroblasts to have a skin regeneration effect, promotes the synthesis of collagen and elastin to have a wrinkle improvement effect, inhibits the synthesis of melanin pigment in melanocytes to have a whitening effect, and promotes the proliferation of dermal papilla cells in the dermis to have hair growth and hair increase effects.

In addition, the present invention provides a cosmetic composition for skin regeneration including the mesenchymal stem cell culture solution.

In the present invention, the "cosmetic composition" is a composition containing the stem cell culture solution, and the formulation may be in any form. For example, cosmetics prepared using the cosmetic composition are creams, packs, lotions, essences, lotions, foundations, makeup bases, and the like, and in order to achieve the object of the present invention, it may be manufactured and commercialized in any form of these formulations but is not limited to the above examples.

In the present invention, the cosmetic composition may further include one or more additives selected from the group consisting of purified water, polyhydric alcohol, surfactant, viscosity modifier, chelating agent, emulsifier, pH adjuster, acid-alkali agent, antioxidant, humectant, brightener, preservative, flavoring agent, fragrance, gelling agent, stabilizer, colorant and pigment.

Further, the present invention provides a cosmetic composition for use in wrinkle improvement and skin elasticity enhancement including the mesenchymal stem cell culture solution.

Further, the present invention provides a cosmetic composition for use in skin whitening including the mesenchymal stem cell culture solution.

Further, the present invention provides a cosmetic composition for use in hair growth or hair increase promotion including the mesenchymal stem cell culture solution.

Further, the present invention provides a method for use in skin regeneration including the step of treating the subject with the mesenchymal stem cell culture solution.

Further, the present invention provides a method for use in wrinkle improvement and skin elasticity enhancement including the step of treating the subject with the mesenchymal stem cell culture solution.

Further, the present invention provides a method for skin whitening including the step of treating the subject with the mesenchymal stem cell culture solution.

Further, the present invention provides a method for hair growth or hair increase promotion including the step of treating the subject with the mesenchymal stem cell culture solution.

Terms not otherwise defined in the present specification have the meanings commonly used in the art to which the present invention pertains.

Hereinafter, the present invention is described in detail by way of Examples. However, the following Examples only illustrate the present invention, and the content of the present invention is not limited by the following Examples.

### Example 1. Mesenchymal stem cell culture and culture solution collection in FBS or hPL-containing medium

### 1.1 Obtaining bone marrow-derived monoclonal mesenchymal stem cells isolated by subfractionation culturing method (SCM)

A 35-year-old male bone marrow donor was anesthetized in the hip area with a local anesthetic, and then the bone marrow was collected by inserting a needle into the hip bone. 10 ml DMEM (Dulbecco's modified eagle's medium, GIBCO-BRL, Life-technologies, MD, USA) containing 20% FBS and 1% penicillin/streptomycin and 3 ml of human bone marrow were added in a 100 mm culture vessel, and they were cultured for 2 hours at 37°C in 5% CO₂ cell incubator. After culturing, only the upper culture solution of the culture vessel was transferred to a new vessel by tilting the culture vessel to one side as much as possible so that the cells attached to the bottom did not fall off as much as possible. After repeating the same process once more, the obtained culture solution was transferred to a culture vessel (Becton Dickinson) coated with collagen on the bottom, and cultured at 37°C for 2 hours. The culture solution was again transferred to a new vessel coated with collagen, and then transferred to a new vessel after 24 hours, and then transferred to a new vessel after 24 hours. Finally, it was transferred to a new vessel after 48 hours. It was visually confirmed that the remaining cells were attached to the bottom of the culture vessel and grew. After about 3 to 5 days, the cells formed a single clone. These single clones were treated with trypsin to separate them. The subfractionation culturing method (Korean Application No. 10-2006-0075676, SCM) was used to obtain the isolated bone marrow-derived monoclonal mesenchymal stem cells.

### 1.2 Mesenchymal stem cell culture and culture solution collection in FBS or hPL-containing medium

Bone marrow-derived monoclonal mesenchymal stem cells isolated by the subfractionation culturing method as in Example 1.1 were inoculated into a low glucose DMEM at a cell density of 1,000 cells/cm² and cultured at 37°C in 5% CO₂ incubator. At this time, the cells were cultured in a medium containing 10% (w/w) fetal bovine serum (FBS) or 4% (w/w) human platelet lysate (hPL) at 37°C in 5% CO₂ cell incubator until the confluency of the cells were 80%. Human platelet lysate (hPL) was purchased from Mill Creek Life Sciences, LLC.

Thereafter, the cells were washed three times with phosphate buffered saline (PBS). The cultured stem cells were transferred to a serum-free medium without antibiotics (gentamicin or penicillin) and phenol red and were cultured for 72 hours. Then, the stem cell-derived culture solution was collected, and the collected culture solution was centrifuged at 3,000 rpm and filtered (Top filter system, Nunc) to produce FBS-conditioned medium (FBS-CM) and hPL-conditioned medium (hPL-CM), respectively. They were used after refrigeration and freezing. As a control, a serum-free medium cultured under the same conditions without cells was used.

### Example 2. Analysis of growth factors present in FBS-CM or hPL-CM using protein array method

Cytokines present in FBS-CM and hPL-CM prepared as in Example 1 were analyzed. The analysis method was performed by using a commercially available assay kit (proteome profiler human XL cytokine array kit, R&D) according to the manual provided with the kit. After color development, LAS4000 (Fuji, Japan) was used for observation.

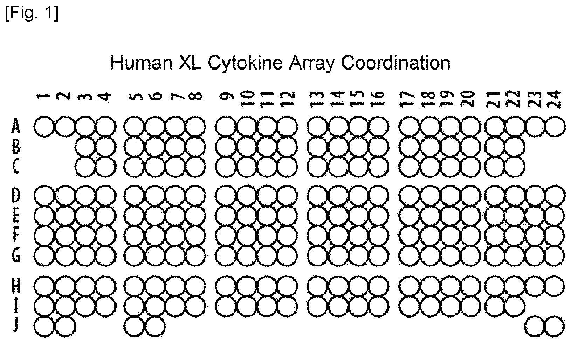

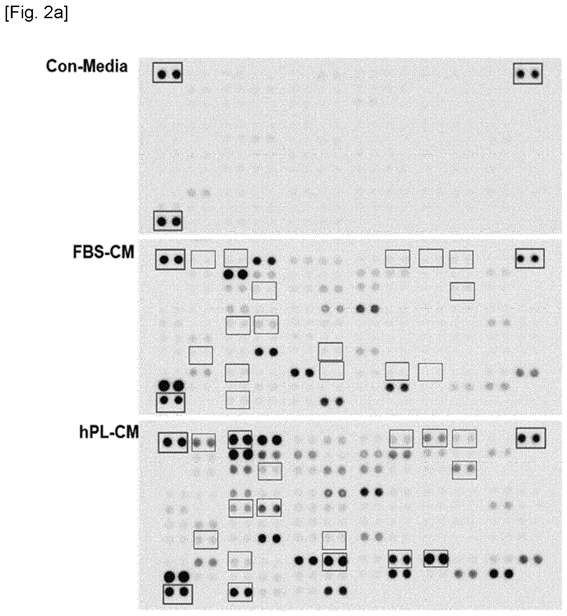

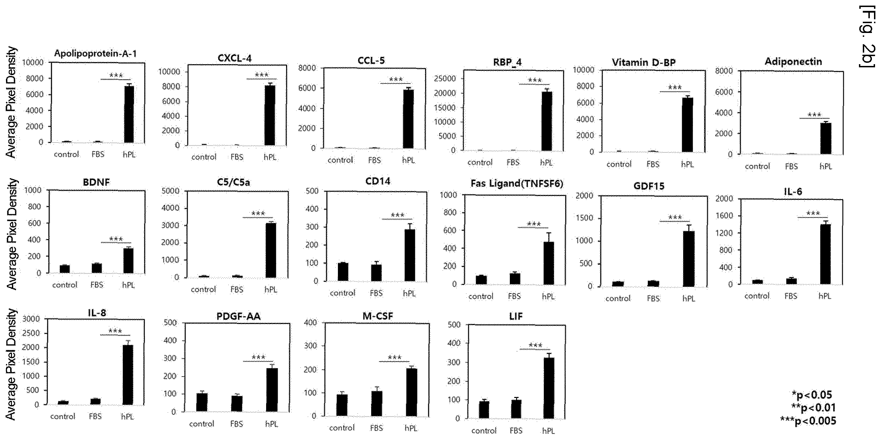

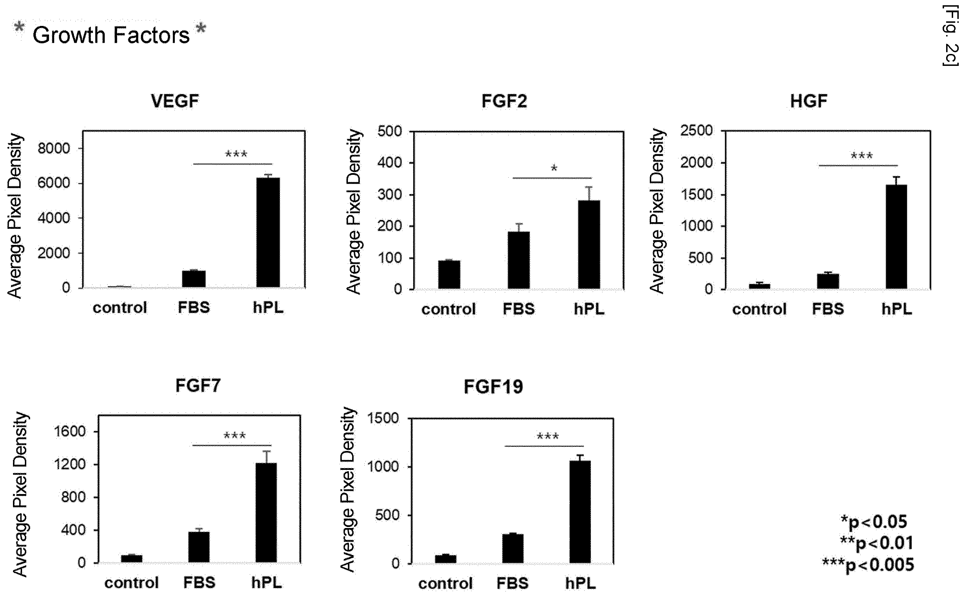

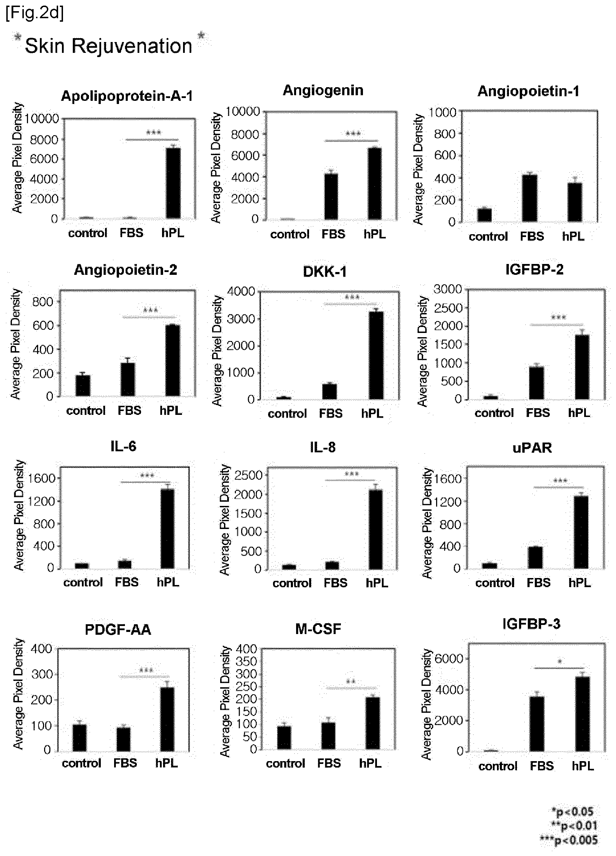

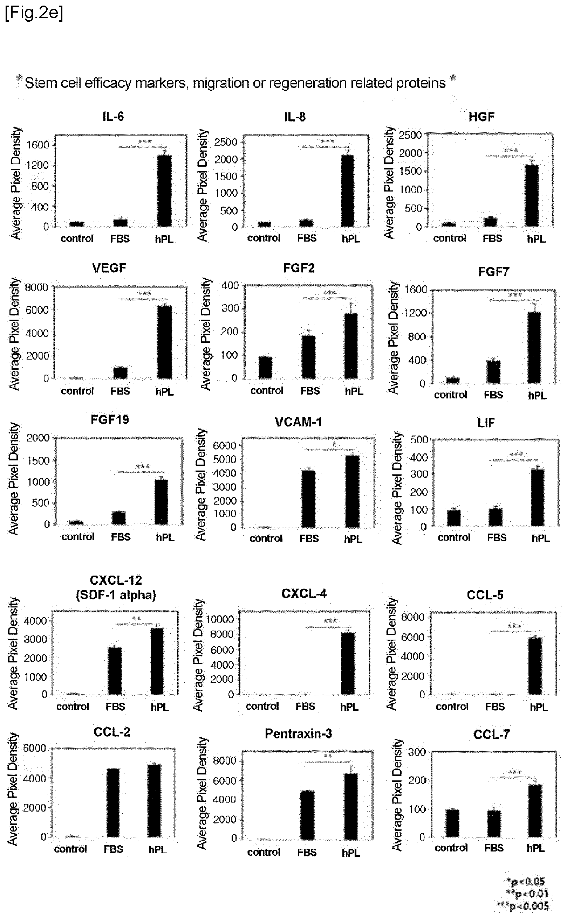

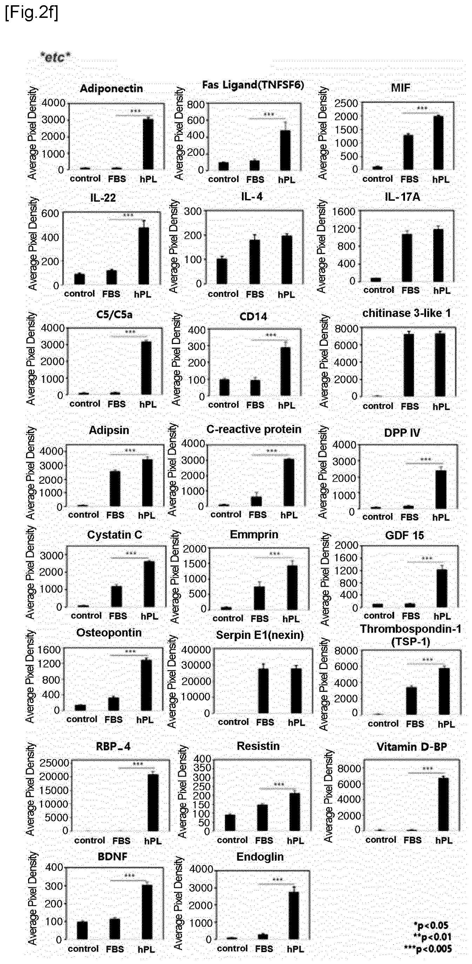

Serum-free DMEM Media cultured under the same conditions without cells was used as a control group. FBS-CM and hPL-CM prepared as in Example 1 were used as an experimental group for comparison. An array map (ARRAY MAP) that can confirm the components contained in each stem cell culture solution is shown in FIG. 1 and Table 1. The relative amount of each growth factor was confirmed by comparing and analyzing spots through the Image J device. The result of the array obtained by the experiment is shown in FIG. 2.

**[Table 1]**

| Cordinate | Subject of Analysis/Controls | Coordinate | Subject of Analysis/Controls | Coordinate | Subject of Analysis/Controls |
|---|---|---|---|---|---|
| A1, A2 | Reference Spots | D13, D14 | IGFBP-3 | G17, G18 | MIP-1α/MIP-1β |
| A3, A4 | Adiponectin | D15, D16 | IL-1α | G19, G20 | MIP-3α |
| A5, A6 | Aggrecan | D17, D18 | IL-1β | G21, G22 | MIP-3β |
| A7, A8 | Angiogenin | D19, D20 | IL-1ra | G23, G24 | MMP-9 |
| A9, A10 | Angiopoietin-1 | D21, D22 | IL-2 | H1, H2 | Myeloperoxidase |
| A11, A12 | Angiopoietin-2 | D23, D24 | IL-3 | H3, H4 | Osteopontin |
| A13, A14 | BAFF | E1, E2 | IL-4 | H5, H6 | PDGF-AA |
| A15, A16 | BDNF | E3, E4 | IL-5 | H7, H8 | PDGF-AB/BB |
| A17, A18 | Complement Component C5/C5a | E5, E6 | IL-6 | H9, H10 | Pentraxin-3 |
| A19, A20 | CD14 | E7, E8 | IL-8 | H11, H12 | PF-4 |
| A21, A22 | CD30 | E9, E10 | IL-10 | H13, H14 | RAGE |
| A23, A24 | Reference Spots | E11, E12 | IL-11 | H15, H16 | RANTES |
| B3, B4 | CD40 ligand | E13, E14 | IL-12p70 | H17, H18 | RBP4 |
| B5, B6 | Chitinase 3-like 1 | E15, E16 | IL-13 | H19, H20 | Relaxin-2 |
| B7, B8 | Complement Factor D | E17, E18 | IL-15 | H21, H22 | Resistin |
| B9, B10 | C-reactive protein | E19, E20 | IL-16 | H23, H24 | SDF-1α |
| B11, B12 | Cripto-1 | E21, E22 | IL-17A | I1, I2 | Serpin E1 |
| B13, B14 | Cystatin C | E23, E24 | IL-18BPa | I3, I4 | SHBG |
| B15, B16 | DKK-1 | F1, F2 | IL-19 | I5, I6 | ST2 |
| B17, B18 | DPP IV | F3, F4 | IL-22 | I7, I8 | TARC |
| B19, B20 | EGF | F5, F6 | IL-23 | I9, I10 | TFF3 |
| B21, B22 | EMMPRIN | F7, F8 | IL-24 | I11, I12 | TfR |
| C3, C4 | ENA-78 | F9, F10 | IL-27 | I13, I14 | TGF-α |
| C5, C6 | Endoglin | F11, F12 | IL-31 | I15, I16 | Thrombospondin-1 |
| C7, C8 | Fas Ligand | F13, F14 | IL-32α/β/γ | I17, I18 | TNF-α |
| C9, C10 | FGF basic | F15, F16 | IL-33 | I19, I20 | uPAR |
| C11, C12 | FGF-7 | F17, F18 | IL-34 | I21, I22 | VEGF |
| C13, C14 | FGF-19 | F19, F20 | IP-10 | J1, J2 | Reference Spots |
| C15, C16 | Flt-3 Ligand | F21, F22 | I-TAC | J5, J6 | Vitamin D-BP |
| C17, C18 | G-CSF | F23, F24 | Kallikrein 3 | J23, J24 | Negative Controls |
| C19, C20 | GDF-15 | G1, G2 | Leptin | | |
| C21, C22 | GM-CSF | G3, G4 | LIF | | |
| D1, D2 | GRO-α | G5, G6 | Lipocalin-2 | | |
| D3, D4 | Growth Hormone | G7, G8 | MCP-1 | | |
| D5, D6 | HGF | G9, G10 | MCP-3 | | |
| D7, D8 | ICAM-1 | G11, G12 | M-CSF | | |
| D9, D10 | INF-γ | G13, G14 | MIF | | |
| D11, D12 | IGFBP-2 | G15, G16 | MIG | | |

As shown in FIG. 2A, it was confirmed that various cytokines and growth factors were present in the stem cell-derived serum-free culture solution. Comparing FBS-CM and hPL-CM, it was confirmed that 32 proteins were detected in FBS-CM and 48 proteins were detected in hPL-CM. It was confirmed that the 48 proteins detected in hPL-CM included apolipoprotein-A-1, chemokine (C-X-C motif) ligand-4 (CXCL-4), chemokine (C-C motif) ligand-5 (CCL-5), retinol binding protein-4 (RBP-4), urokinase receptor (uPAR), vitamin D-BP, vascular endothelial growth factor (VEGF), fibroblast growth factor 2 (FGF2), hepatocyte growth factor (HGF), fibroblast growth factor 7 (FGF7), fibroblast growth factor 19 (FGF19), angiogenin, angiopoietin-1, angiopoietin-2, Dickoff related protein 1 (DKK-1), insulin like growth factor binding protein-2 (IGFBP-2), interleukin-6 (IL-6), interleukin-8 (IL -8), platelet derived growth factor-AA (PDGF-AA), macrophage colony stimulating factor (M-CSF), insulin like growth factor binding protein-3 (IGFBP-3), vascular cell adhesion molecule-1 (VCAM-1), leukemia inhibitory factor (LIF), chemokine (C-X-C motif) ligand-12 (CXCL-12), chemokine (C-C motif) ligand-2 (CCL-2), pentraxin-3, chemokine (C-C motif) ligand-7 (CCL-7), adiponectin, Fas ligand (TNF SF6), macrophage migration inhibitory factor (MIF), interleukin-22 (IL-22), interleukin-4 (IL-4), interleukin-17A (IL-17A), complement component 5/complement component 5a (C5/C5a), cluster of differentiation 14 (CD14), chitinase 3-like 1, adipsin, C-reactive protein, dipeptidyl peptidase IV (DPP IV), cystatin C, emmprin, growth/differentiation factor 15 (GDF15), osteopontin, serpin E1 (nexin), thrombospondin-1 (TSP-1), resistin, brain derived neurotrophic factor (BDNF) and endoglin. Among the 48 proteins, a representative protein expressed only in hPL-CM compared to FBS-CM is shown in FIG. 2B, a growth factor-related protein is shown in FIG. 2C, a skin rejuvenation-related protein is shown in FIG. 2D, stem cell efficacy markers, migration or regeneration-related proteins are shown in FIG. 2E, and other proteins expressed are shown in FIG. 2F.

### Example 3. Comparison of growth factor amount of FBS-CM or hPL-CM

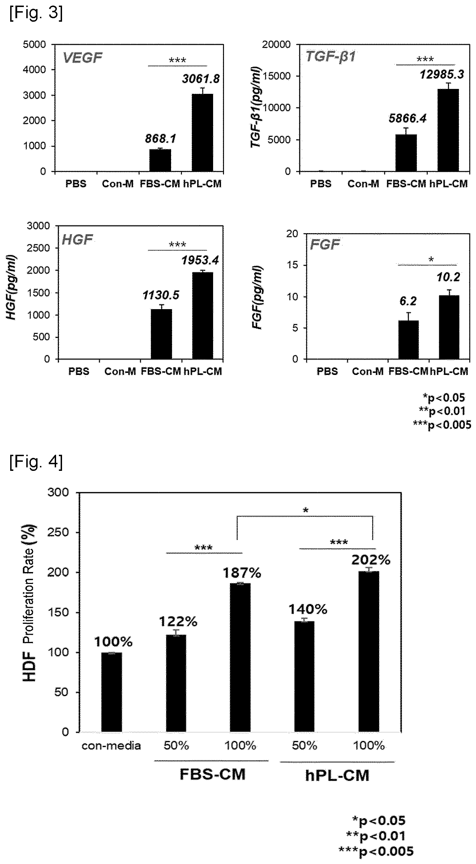

The results of Example 2 confirmed that more types of growth factors and proteins were included in the hPL-CM of the present invention. For a more accurate comparison, the amounts of representative growth factors present in FBS-CM or hPL-CM prepared in the same manner as in Example 1 were compared, and the results are shown in FIG. 3.

As shown in FIG. 3, it was confirmed that the amounts of VEGF, TGF-beta1, HGF, and FGF were increased in hPL-CM than in FBS-CM. Specifically, in the case of VEGF, hPL-CM contained 3.5 times or more than FBS-CM. In the case of TGF-beta1, hPL-CM contained 2.2 times or more than FBS-CM. In the case of HGF, hPL-CM contained 1.7 times or more than FBS-CM. In the case of FGF, hPL-CM contained 1,6 times or more than FBS-CM. In other words, it was confirmed once again that the amount of the growth factors present in the stem cell culture solution could be increased by culturing the mesenchymal stem cells in the hPL-containing medium.

### Example 4. Comparison of human dermal fibroblast proliferation effect of FBS-CM or hPL-CM

The results of Examples 2 and 3 confirmed that the hPL-CM of the present invention contained more types and high concentrations of growth factors and proteins. Accordingly, it was attempted to determine whether there is a difference in skin improvement efficacy. Specifically, a group in which human dermal fibroblasts (HDF, purchased from Lonza) were treated with a serum-free medium for 24 hours and was used as a control group. As an experimental group, human dermal fibroblasts were treated with 100% FBS-CM stock solution prepared in the same way as in Example 1, 50% FBS-CM diluted solution in which the FBS-CM stock solution was diluted 50% with serum-free medium, 100% hPL-CM stock solution prepared in the same way as in Example 1, and 50% hPL-CM diluted solution in which the hPL-CM stock solution was diluted 50% with serum-free medium, respectively. The proliferation-promoting effect of human dermal cells in each control group and experimental group was confirmed through CCK-8, and the results are shown in FIG. 4.

As shown in FIG. 4, it was confirmed that the proliferation-promoting effect of human dermal fibroblasts increased by 100% or more in the hPL-CM-treated group compared to the control group. The skin regeneration effect of hPL-CM was confirmed by confirming the tendency that the proliferation promoting effect of human dermal fibroblasts was higher in the hPL-CM-treated group than in the FBS-CM-treated group.

### Example 5. Collagen synthesis effect of FBS-CM or hPL-CM

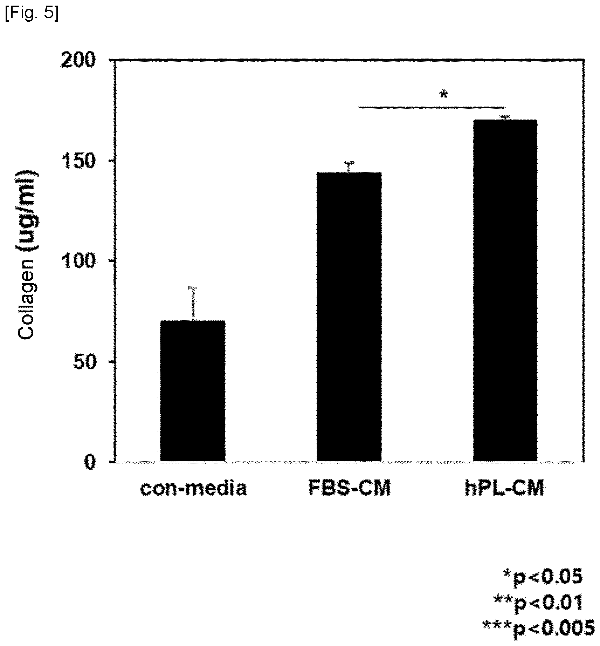

In this example, in order to confirm the effectiveness of the stem cell culture solution as a skin aging inhibitor, the collagen synthesis rate was confirmed using human-derived skin fibroblasts. Specifically, after culturing human dermal fibroblasts in a 6-well plate for 24 hours, FBS-CM and hPL-CM, or a control culture solution (serum-free DMEM media cultured under the same conditions without cells) prepared in the same manner as in Example 1 were treated in 1,000 µl. They were cultured for 72 hours. The amount of collagen newly synthesized and secreted by human dermal fibroblasts was measured using a collagen kit (Sircol TM collagen assay kit), and the results are shown in FIG. 5.

As shown in FIG. 5, it was confirmed that the synthetic amount of collagen of the hPL-CM-treated group was 170.0 µg/ml, which was higher by about 18% compared to 143.6 µg/ml in the FBS-CM-treated group. That is, it was confirmed that hPL-CM was superior to FBS-CM in collagen synthesis, which helps skin elasticity, skin regeneration, and skin wrinkle improvement.

### Example 6. Elastin synthesis effect of FBS-CM or hPL-CM

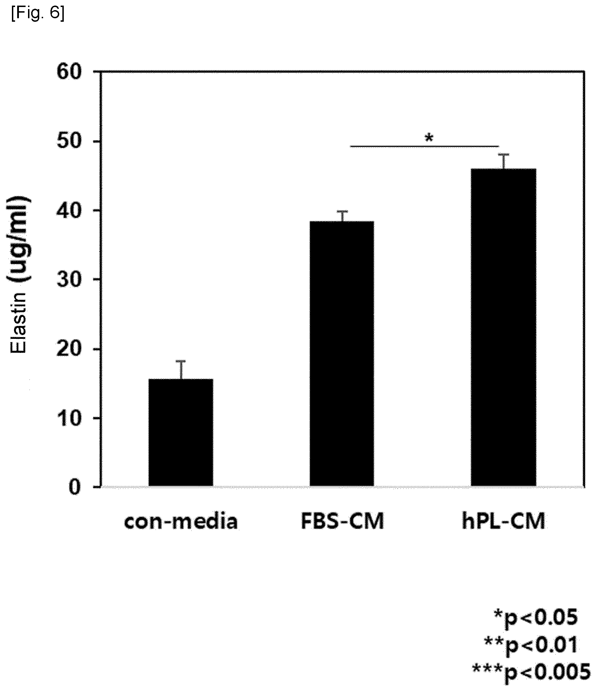

Skin elasticity is induced by elastic fibers consisting of elastin present in the dermis layer. These elastic fibers exist together with collagen, and the elasticity of the skin can be maintained in a state where elastin and collagen are sufficiently present. An experiment was conducted to confirm the elastin synthesis promoting effect of FBS-CM or hPL-CM. Specifically, after seeding human dermal fibroblasts in a 24-well plate, 24 hours later, when all cells were attached, all culture medium was removed. Then, 500 µl of FBS-CM, hPL-CM or serum-free medium (control group) was added into each well. After culturing for 72 hours, the amount of elastin was measured, and the results are shown in FIG. 6.

As shown in FIG. 6, the results of comparing the amount of elastin synthesis confirmed that the amount of elastin synthesis of the FBS-CM-treated group and the hPL-CM-treated group, respectively, were 38.3 µg/ml and 46.0 µg/ml. It was confirmed that the amount of elastin synthesis was measured higher by about 20% in the hPL-CM-treated group. In other words, it was confirmed that hPL-CM was more effective in elastin synthesis, which helps skin elasticity, skin regeneration, and skin wrinkle improvement.

### Example 7. Confirmation of skin whitening effect using 3D artificial skin

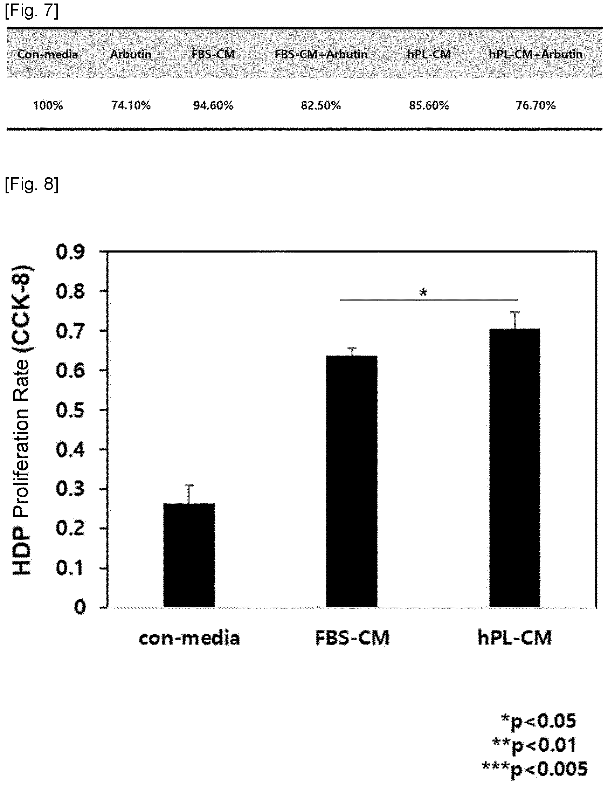

In order to confirm the skin whitening effect of the stem cell culture solution, a decrease in the amount of melanin was confirmed using artificial skin. MEL-300-Melanoderm tissues (MarTek Co. Kr) were used as 3D artificial skin. They were cultured at 37°C and 5% CO₂ incubator conditions, and the results were observed. Specifically, a serum-free culture solution-treated group was used as a control group. A group treated with 200 µl of arbutin at a concentration of 1,000 µg/ml, a group treated with 200 µl of FBS-CM prepared in Example 1, a group co-treated with 100 µl of arbutin at a concentration of 1000 µg/ml and 100 µl of FBS-CM prepared in Example 1, a group treated with 200 µl of hPL-CM prepared in Example 1, and a group co-treated with 100 µl of arbutin at a concentration of 1,000 µg/ml and 100 µl of hPL-CM prepared in Example 1 were used as experimental groups. The artificial skin placed on the transwell was repeatedly treated with FBS-CM, hPL-CM or arbutin as set above once every 2 days and cultured for 21 days. Then the amount of melanin synthesis was measured, and the measurement result is shown in FIG. 7.

As shown in FIG. 7, when the amount of melanin synthesis of the control group was 100%, the amount of melanin synthesis was decreased to 74.1% in arbutin as the positive control group, and the FBS-CM-treated group and the hPL-CM-treated group were decreased to 94.6% and 85.6%, respectively. When arbutin, a positive control, and FBS-CM and hPL-CM were co-treated (combination), the amount of melanin synthesis was further reduced to 82.5% for arbutin/FBS-CM and 76.7% for arbutin/hPL-CM. That is, it was confirmed that hPL-CM showed a higher whitening effect than FBS-CM through the amount of melanin synthesis of 3D artificial skin showing skin whitening effect.

### Example 8. Confirmation of hair growth efficacy of FBS-CM or hPL-CM

Hair growth may be induced by the generation and proliferation of dermal papilla cells. Therefore, in order to confirm the hair growth efficacy of the stem cell culture solution according to the culture conditions, it was checked whether it affects growth, that is, proliferation using primary human dermal papilla cells. Specifically, the FBS-CM-treated group prepared in the same way as in Example 1 or the hPL-CM-treated group prepared in the same way as in Example 1 was used as the experimental group, and the serum-free medium-treated group was set as the control group. 24 hours after seeding human papilla cells (purchased by PromoCell) in a 96-well plate, when all the cells were attached, 200 µl of FBS-CM, hPL-CM or serum-free medium was added to each well, and then cultured for 48 hours. The proliferation promoting effect on human papilla cells was confirmed through CCK-8, and the results are shown in FIG. 8.

As shown in FIG. 8, it was confirmed that when the stem cell culture solution was treated, the proliferation promoting effect of the hPL-CM-treated group was increased 2.6 times or more compared to the control group, and the proliferation promoting effect of the hPL-CM-treated group was higher by about 20% than the FBS-CM-treated group. Thus, the hair growth effect of hPL-CM was confirmed.

### Example 9. Comparison of human dermal fibroblast proliferation effects according to culture conditions and isolation methods

Mesenchymal stem cell culture solution prepared by the same manner as in Example 1.2 for bone marrow-derived monoclonal mesenchymal stem cells isolated by gradient centrifugation method (GCM) (hereinafter, referred to as GCM conditioned medium, "GCM-CM") and the stem cell culture solution isolated by the subfractionation culturing method of Example 1 (hereinafter, referred to as "SCM-CM") were compared in efficacy.

### 9.1 Setting experimental group and control group

The mesenchymal stem cells isolated by the GCM separation method were cultured in an FBS or hPL-containing medium in the same manner as in Example 1.2, and then transferred to a serum-free medium and cultured to obtain a mesenchymal stem cell culture solution (hereinafter, referred to as "GCM-FBS-CM-treated group" or "GCM-hPL-CM-treated group"). The GCM-FBS-CM-treated group and GCM-hPL-CM-treated group were set as experimental groups. Bone marrow-derived monoclonal mesenchymal stem cells isolated by subfractionation culturing method were cultured in an FBS or hPL-containing medium as in Example 1, and then transferred to a serum-free medium and cultured to obtain a mesenchymal stem cell culture solution (hereinafter, referred to as "SCM-FBS-CM-treated group" or "SCM-hPL-CM-treated group"). The SCM-FBS-CM-treated group and SCM-hPL-CM-treated group were set as experimental groups. A serum-free medium-treated group was set as a control group.

### 9.2 Measurement of the proliferation effect of human dermal fibroblasts

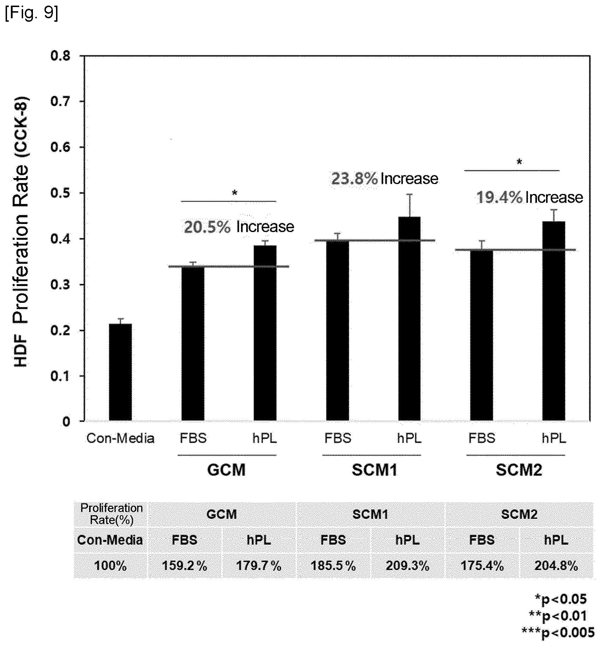

24 hours After seeding human dermal fibroblasts in a 96-well plate, when all cells were attached, all culture medium was removed. Then, 200 µl of the stem cell culture solution or serum-free medium as set in Example 9.1 above was added to each well, and they were cultured for 48 hours. The proliferation promoting effect of human dermal fibroblasts was confirmed through CCK-8, and the results are shown in FIG. 9.

As shown in FIG. 9, it was confirmed that the proliferative effect of human dermal fibroblasts was higher in the hPL culture condition than in the FBS culture condition in the same cell line. It was confirmed that the proliferation rate was higher in the two cell lines cultured by the SCM separation method than by the GCM separation method.

### Example 10. Comparison of VEGF amount according to culture conditions and separation methods

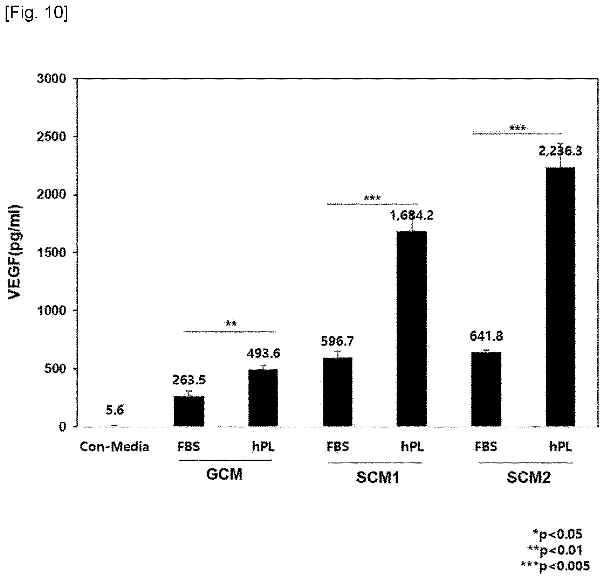

The efficacy of the culture solution of mesenchymal stem cells isolated by the GCM separation method and the stem cell culture solution isolated by the subfractionation culturing method of Example 1 was further compared. Specifically, the amount of VEGF, a representative growth factor, was compared by the same method as in Example 2 for the control group prepared in Example 9.1 and the experimental group, GCM-FBS-CM group, GCM-hPL-CM group, SCM-FBS-CM group, and SCM-hPL-CM group, and the results are shown in FIG. 10.

As shown in FIG. 10, the results of comparing the amount of VEGF in the stem cell culture solution confirmed that the amount of VEGF in the mesenchymal stem cell culture solution cultured in the hPL-containing medium was higher than that in the mesenchymal stem cell culture solution cultured in the FBS-containing medium from the mesenchymal stem cell line isolated in the same manner. It was confirmed that the amount of VEGF was higher in the two cell lines cultured by the SCM separation method than in the case cultured by the GCM separation method.

Above, specific parts of the present invention have been described in detail, for those of ordinary skill in the art, these specific descriptions are only preferred embodiments, and it is clear that the scope of the present invention is not limited thereby. Accordingly, the substantial scope of the present invention is defined by the appended claims and their equivalents.

The composition according to the present invention may be formulated in various forms depending on the purpose. The following exemplifies several formulation methods containing the composition according to the present invention as an active ingredient, but the present invention is not limited thereto.

### Formulation example 1: lotion

A formulation example of the lotion among the cosmetics containing the mesenchymal stem cell culture solution of the present invention is as follows.
Ingredients (amount, unit: wt%)
Mesenchymal stem cell culture solution of the present invention 2.0
Glycerin 5.0
1.3-Butylene Glycol 3.0
PEG 1500 1.0
Allantoin 0.1
DL-Panthenol 0.3
EDTA-2Na 0.02
Benzophenone-9 0.04
Sodium Hyaluronate 5.0
Ethanol 10.0
Octyldodeceth-16 0.2
Polysorbate 20 0.2
Preservatives, Fragrances, and Colors Trace amounts
Distilled water residual
Total 100

### Formulation Example 2: Cream

A prescription example of a cream among cosmetics containing the mesenchymal stem cell culture solution of the present invention is as follows.
Ingredients (amount, unit: wt%)
Mesenchymal stem cell culture solution of the present invention 2.0
Lipophilic Monostearate Glycerin 2.0
Cetearyl alcohol 2.2
Stearic acid 1.5
Beeswax 1.0
Polysorbate 60 1.6
Sorbitan Stearate 0.6
Hardened vegetable oil 1.0
Squalene 3.0
Mineral Oil 5.0
Trioctanoin 5.0
Dimethicone 1.0
Sodium Magnesium Silicate 0.1
Glycerin 5.0
Betaine 3.0
Triethanolamine 1.0
Sodium Hyaluronate 4.0
Preservatives, Fragrances, and Colors Trace amounts
Distilled water residual
Total 100

### Formulation Example 3: Essence

Prescription examples of the essence in the cosmetics containing the mesenchymal stem cell culture solution of the present invention are as follows.
Ingredients (amount, unit: wt%)
Mesenchymal stem cell culture solution of the present invention 2.0
Glycerin 10.0
Betaine 5.0
PEG 1500 2.0
Allantoin 0.1
DL-Panthenol 0.3
EDTA-2Na 0.02
Benzophenone-9 0.04
Hydroxyethyl Cellulose 0.1
Sodium Hyaluronate 8.0
Carboxyvinyl Polymer 0.2
Triethanolamine 0.18
Octyldodecanol 0.3
Octyldodeceth-16 0.4
Ethanol 6.0
Preservatives, Fragrances, and Colors Trace amounts
Distilled water residual
Total 100

Above, specific parts of the present invention have been described in detail, for those of ordinary skill in the art, these specific descriptions are only preferred embodiments, and it is clear that the scope of the present invention is not limited thereby. Accordingly, the substantial scope of the present invention is defined by the appended claims and their equivalents.

## Claims

1. A method for producing a mesenchymal stem cell culture solution rich in growth factors and cytokines, the method comprising steps of:
1) culturing mesenchymal stem cells in a medium containing human platelet lysate (hPL);
2) obtaining the cultured stem cell and culturing the stem cells in a serum-free medium; and
3) collecting and filtering the mesenchymal stem cell-derived culture solution to obtain a stem cell culture solution.

2. The method of claim 1, wherein the medium containing hPL is a medium containing 1% to 10% (w/w) hPL.

3. The method of claim 1, wherein the mesenchymal stem cells are bone marrow-derived monoclonal mesenchymal stem cells isolated by a subfractionation culturing method (SCM) including steps of:
1) culturing bone marrow isolated from a subject;
2) transferring only supernatant from step 1) to a new vessel and culturing the same;
3) isolating only supernatant from step 2), then culturing for 1 hour to 3 hours at 35°C to 39°C in a culture vessel, then repeating 2 to 3 times culture for 12 hours to 36 hours at 35°C to 39°C, then culturing for 24 hours to 72 hours at 35°C to 39°C, wherein the culture is repeated such that the supernatant is transferred to a new culture vessel each time; and
4) obtaining monoclonal stem cells from the final culture vessel.

4. The method of claim 1, wherein the mesenchymal stem cells in step 1) are inoculated at a density of 50 cells/cm² to 1,000 cells/cm².

5. The method of claim 1, wherein the growth factor and cytokine are any one or more selected from the group consisting of apolipoprotein-A-1, chemokine (CXC motif) ligand-4 (CXCL-4), chemokine (C-C motif) ligand-5 (CCL-5), retinol binding protein-4 (RBP-4), vitamin D-BP, adiponectin, brain-derived neurotrophic factor (BDNF), complement component 5/complement component 5a (C5/C5a), cluster of differentiation 14 (CD14), Fas ligand (TNF SF6), growth/differentiation factor 15 (GDF15), interleukin-6 (IL-6), interleukin-8 (IL-8), platelet-derived growth factor-AA (PDGF-AA), macrophage colony stimulating factor (M-CSF) and leukemia inhibitory factor (LIF).

6. The method of claim 5, wherein the growth factor and cytokine further include any one or more selected from the group consisting of vascular endothelial growth factor (VEGF), fibroblast growth factor 2 (FGF2), hepatocyte growth factor (HGF), fibroblast growth factor 7 (FGF7), fibroblast growth factor 19 (FGF19), angiogenin, angiopoietin-1, angiopoietin-2, Dickkopf related protein 1 (DKK-1), insulin like growth factor binding protein-2 (IGFBP-2), insulin like growth factor binding protein-3 (IGFBP-3), vascular cell adhesion molecule-1 (VCAM-1), CXCL-12(chemokine (CXC motif) ligand-12), chemokine (C-C motif) ligand-2 (CCL-2), pentraxin-3, chemokine (C-C motif) ligand-7 (CCL-7), macrophage migration inhibitory factor (MIF), interleukin-22 (IL-22), interleukin-4 (IL-4), interleukin-17A (IL-17A), chitinase 3-like 1, adipsin, C-reactive protein, dipeptidyl peptidase IV (DPP IV), cystatin C, emmprin, osteopontin, serpin E1 (nexin), thrombospondin-1 (TSP-1), resistin, urokinase receptor (uPAR) and endoglin.

7. A method for producing a mesenchymal stem cell culture solution rich in growth factors and cytokines for preparing a cosmetic composition for use in skin regeneration, wrinkle improvement, skin whitening, hair growth or hair increase, the method comprising steps of:
1) culturing mesenchymal stem cells in a medium containing human platelet lysate (hPL);
2) obtaining the cultured stem cells and culturing them in a serum-free medium; and
3) collecting and filtering the mesenchymal stem cell-derived culture solution to obtain a stem cell culture solution.

8. A mesenchymal stem cell culture solution rich in growth factors and cytokines prepared by the method of any one of claims 1 to 7.

9. A cosmetic composition for use in skin regeneration comprising the mesenchymal stem cell culture solution of claim 8.

10. A cosmetic composition for use in wrinkle improvement and skin elasticity enhancement comprising the mesenchymal stem cell culture solution of claim 8.

11. A cosmetic composition for use in skin whitening comprising the mesenchymal stem cell culture solution of claim 8.

12. A cosmetic composition for use in hair growth or hair increase promotion comprising the mesenchymal stem cell culture solution of claim 8.

13. A method selected from the group consisting of skin regeneration; wrinkle improvement and skin elasticity enhancement; skin whitening; and hair growth or hair increase promotion, the method comprising a step of treating a subject with the mesenchymal stem cell culture solution of claim 8.
